# FASCICULE DE BREVET EUROPEEN

(11) **EP 2 756 839 B1**
(45) Date de publication et mention de la délivrance du brevet: **26.06.2019**
(21) Numéro de dépôt: 14151803.5
(22) Date de dépôt: 20.01.2014
(51) Int. Cl.: A61K 8/73, A61Q 19/00, A61K 35/08, A61K 9/00, A61K 47/38, A61K 8/04, A61K 8/96, A61K 9/06, A61P 1/02, A61P 17/00, A61P 21/00

(54) **Composition sous forme de gel à base d'eau de mer, notamment pour une utilisation en massage**
Zusammensetzung in Form eines Gels auf Meerwasserbasis, insbesondere zum Einsatz bei Massagen
Composition in the form of a seawater gel, especially for use in massage

(30) Priorité: 21.01.2013 FR 1350518
(43) Date de publication de la demande: 23.07.2014
(73) Titulaire: H2-Zoow SAS, 75008 Paris (FR)
(72) Inventeur: MAUGENDRE, Renée-Jane, 35400 Saint-Malo (FR)
(74) Mandataire: Cabinet Le Guen Maillet

(56) Documents cités:
- EP-A1- 1 435 229
- JP-A- 2000 273 033
- US-A- 3 341 319
- US-A- 3 889 007
- DATABASE GNPD [Online] MINTEL; octobre 2007 (2007-10), "Body Scrub", XP002714297, Database accession no. 797743
- DATABASE GNPD [Online] MINTEL; mars 2011 (2011-03), "Toner", XP002714298, Database accession no. 1478246
- DATABASE GNPD [Online] MINTEL; mai 2010 (2010-05), "Sun Lotion SPF 35/PA++", XP002714299, Database accession no. 1331584
- DATABASE WPI Week 201050 Thomson Scientific, London, GB; AN 2010-D18664 XP002714300, & KR 2010 0024624 A (GANGWON DEEP SEA WATER CO LTD) 8 mars 2010 (2010-03-08)
- DATABASE WPI Week 200913 Thomson Scientific, London, GB; AN 2009-E59143 XP002714301, & KR 2008 0100507 A (KIM K J) 19 novembre 2008 (2008-11-19)
- DATABASE WPI Week 200913 Thomson Scientific, London, GB; AN 2009-E59144 XP002714302, & KR 2008 0100506 A (KIM K J) 19 novembre 2008 (2008-11-19)
- CHUNYU CHANG ET AL: "Cellulose-based hydrogels: Present status and application prospects", CARBOHYDRATE POLYMERS, APPLIED SCIENCE PUBLISHERS, LTD. BARKING, GB, vol. 84, no. 1, 8 décembre 2010 (2010-12-08), pages 40-53, XP028151866, ISSN: 0144-8617, DOI: 10.1016/J.CARBPOL.2010.12.023 [extrait le 2010-12-15]

## Description

La présente invention concerne une composition sous forme de gel à base d'eau de mer, notamment pour une utilisation en massage d'une partie du corps.

L'eau de mer est connue pour ses qualités thérapeutiques depuis l'antiquité. Beaucoup de livres et de publications ont répertorié et décrit ses vertus sur la santé. La thalassothérapie est un des exemples de l'utilisation de l'eau de mer en traitement thérapeutique. A titre d'exemple, l'eau de mer y est utilisée pour les soins de pathologies telles que l'eczéma et le psoriasis, et la remise en forme.

Dans le cas de traumatismes et de pathologies musculo tendineuses ou d'entorses, il est généralement recommandé par les kinésithérapeutes de pratiquer la marche dans l'eau de mer et de même par les vétérinaires concernant les chevaux.

Les traitements permettant aujourd'hui de bénéficier des vertus de l'eau de mer comprennent des bains, des douches, des pulvérisations par jets ou des hydro-massages, dans les établissements spécialisés.

Cependant, il pourrait être intéressant de pouvoir procéder à des applications locales et prolongées de l'eau de mer permettant ainsi de mieux cibler ces applications à une zone concernée et sans avoir, de plus, recours à du matériel spécifique et encombrant.

On connait le document JP 2000-273033 qui décrit des compositions sous forme de gels contenant des sels inorganiques et des principes actifs. Les compositions sont gélifiées à l'aide de garraghénanes et de tréhalose. Il est évoqué un gel contenant entre 0.1 et 85% d'eau de mer.

Il demeure aujourd'hui encore un besoin de trouver une solution pour formuler des gels contenant plus de 90% d'eay de mer.

Le but de la présente invention est de pallier ce problème en proposant une composition pharmaceutique ou cosmétique, sous forme de gel qui se caractérise en ce qu'elle comprend au moins 90% d'eau de mer, en poids par rapport au poids total de la composition, et de l'hydroxyéthyl méthyl cellulose à titre d'agent gélifiant.

L'eau de mer sous forme d'un gel se trouve alors facilement applicable sur des zones localisées du corps humain ou animal. De plus, la formulation sous forme de gel permet une application de l'eau de mer en tant que gel de massage. Il est également possible de l'appliquer en cataplasme ou en enveloppement.

Selon l'invention, la composition sous forme de gel est hypertonique ou isotonique.

L'eau de mer utilisée dans le cadre de l'invention est une eau de mer naturelle puisée sur un site propice à l'extraction de celle-ci (en profondeur) et dont la concentration massique en chlorure de sodium est comprise entre 28 et 36 g/L. Préférentiellement, l'eau de mer est puisée à marée haute en baie de CANCALE, lieu idéal où se produisent les plus grandes marées du monde (marnage de plus de 12 mètres) et dans une zone balayée par de forts courants marins.

Cette zone est sous la surveillance d'IFREMER, qui, par son réseau R.N.O., assure en permanence une surveillance et un contrôle qualité, selon les normes et directives européennes en vigueur, compte tenu de la présence de nombreux bassins ostréicoles.

Selon un premier mode de réalisation, la composition sous forme de gel comprend au moins 95%, plus préférentiellement encore au moins 98%, en poids par rapport au poids total de la composition.

Préférentiellement selon ce premier mode de réalisation, l'eau de mer est stérilisée à froid par filtration à 0,2µ. La radio stérilisation ou la stérilisation à chaud sont exclues afin de conserver toutes les caractéristiques physicochimiques de l'eau de mer et notamment les oligo éléments. Ainsi, dans ce mode de réalisation, la composition du gel est hypertonique. Elle présentera avantageusement une concentration en chlorure de sodium d'au moins 23 g/L. Le gel hypertonique produit un effet décongestionnant lié à l'effet osmotique direct de l'eau de mer, et cet effet est d'autant plus important que la concentration en eau de mer est élevée.

Dans le cadre de l'invention, l'hydroxyéthyl méthyl cellulose porte le numéro CAS 9032-42-2.

Préférentiellement, la composition comprend entre 1 et 2%, plus préférentiellement 1,5 %, de l'agent gélifiant, en poids par rapport au poids total de la composition.

Selon un mode de réalisation de l'invention, la composition sous forme de gel comprend à titre de solvant de l'eau distillée.

L'eau distillée permet de compléter la formulation mais également, selon les modes de réalisation, de diluer l'eau de mer de manière à obtenir la concentration en chlorure de sodium désirée.

Selon un mode de réalisation de l'invention, la composition sous forme de gel comprend au moins un agent de conditionnement de la peau tel qu'un agent hydratant, un agent bactériostatique et/ou fongistatique.

Préférentiellement, ledit agent de conditionnement de la peau est choisi parmi le pentylène glycol, le caprylyl glycol, décylène glycol ou un mélange des trois. Un tel mélange de ces trois composants est par exemple le mélange vendu sous le nom commercial Microcare Emollient DCP ou des agents de conditionnement à propriétés fongistatiques et bactériostatiques telles que les huiles essentielles.

Préférentiellement, la composition comprend entre 0,2 et 1%, dudit agent de conditionnement de la peau, en poids par rapport au poids total de la composition.

Selon un mode de réalisation de l'invention, la composition sous forme de gel présente un pH compris entre 5 et 6, préférentiellement un pH de 5,5 plus ou moins 0,2 unité de pH. Cette gamme de pH permet une très bonne tolérance au niveau de la peau. Le pH est régulé par un agent de régulation du pH tel que l'acide lactique. La composition en comprend entre 0,01 et 0,02%.

Selon un mode de réalisation préféré de l'invention, la composition ne contient pas d'autre agent actif que l'eau de mer.

L'invention concerne encore une composition sous forme de gel telle que décrite précédemment pour son utilisation en tant que composition pharmaceutique ou de médicament, en particulier pour son utilisation dans le traitement des traumatismes et pathologies musculo-tendineuses, plus particulièrement les entorses, les courbatures, les contractures musculaires, chez l'homme ou l'animal.

En particulier, la composition sous forme de gel selon l'invention s'avère très efficace dans le traitement et le rétablissement post traumatique des pathologies, telles que des entorses ou des tendinites, des membres inférieurs notamment du cheval, tels que les genoux, les jarrets, les boulets et les tendons.

L'invention concerne encore un dispositif médical comprenant une composition sous forme de gel telle que décrite précédemment. Un tel dispositif médical peut être tout applicateur tel qu'un tube ou un dispositif muni d'une pompe.

L'invention concerne encore l'utilisation d'une composition sous forme de gel telle que décrite précédemment en massage, cataplasme ou enveloppement.

L'invention concerne encore une composition sous forme de gel telle que décrite précédemment pour son utilisation dans le traitement ou la prévention de la sensibilité des gencives, du parodonte, en particulier des gingivites et des parodontites.

L'invention concerne encore une composition sous forme de gel telle que décrite précédemment pour son utilisation dans le traitement ou la prévention des sécheresses et/ou rougeurs cutanées du corps et/ou du visage.

Les caractéristiques de l'invention mentionnées ci-dessus, ainsi que d'autres, apparaîtront plus clairement à la lecture de la description suivante d'un exemple de réalisation.

### Exemple 1 : Gel à base d'eau de mer hypertonique :

La composition d'eau de mer sous forme de gel est préparée à partir des ingrédients indiqués dans le Tableau 1 :

**Tableau 1**

| **INCI** | **%** | **FONCTION** | **N°CAS** |
|---|---|---|---|
| Maris aqua (eau de mer) | 97,475 | Principe actif | / |
| Methyl Hydroxyethylcellulose | 1,5 | Agent gélifiant/stabilisant/agent de viscosité | 9032-42-2 |
| Pentylene Glycol | 0,5 | Conditionnement peau /Hydratant | 5343-92-0 |
| Caprylyl Glycol | 0,3 | Conditionnement peau /Hydratant | 1117-86-8 |
| Decylene glycol | 0,2 | Conditionnement peau | 214-288-2 |
| Lactic Acid | 0,02 | Régulateur pH/Hydratant | 50-21-5 |
| aqua(water) | 0,005 | solvant | 7732-18-5 |
| **TOTAL (%)** | **100,000** | | |

| | | | |
|---|---|---|---|
| pH = 5,5 viscosité 32 000 centipoise | | | |

L'eau de mer n'étant pas diluée dans la composition, le gel est hypertonique avec une concentration en chlorure de sodium proche de celle de l'eau de mer (28-36 g/L).

La composition est formulée comme suit :
- eau de mer pesée,
- ajout des pentylène glycol, caprylyl glycol, décylène glycol,
- ajout du méthyl hydroxyéthylcellulose (STRUCTURE CEL 12000 M Polymer - Société AkzoNobel) à froid,
- régulation du pH par ajout de l'acide lactique.

La composition sous forme de gel peut être utilisée en tant que gel de massage ou encore en tant que gel de soin appliqué en cataplasme sur une zone précise du corps humain. Elle peut être utilisée dans tout traitement connu dans la littérature utilisant de l'eau de mer. A ces fins, la composition sera formulée de manière à contenir une eau de mer isotonique ou hypertonique.

Différentes applications de la composition formulée selon l'exemple 1 ont été testées dans ce qui suit.

### 1) Application en massage

- Nombre de sujets inclus : 20 sujets de 21 à 67 ans (Age moyen = 50 ans), dont 55% (n = 11) > 50 ans; 17 femmes et 3 hommes
- Age : - Phototype : de I à IV
- Type de peau sur le site d'application :
   - Bras : peau sèche (60%, n= 12), peau normale (40%, n = 8)
   - Jambes : peau sèche (80%, n = 16), peau normale (20%, n = 4)
- 65 % du panel (n = 13) étant sportifs
- Utilisateurs réguliers/occasionnels de produits similaires

### Application du produit :

□ Application au centre d'investigation le premier jour (J1) par les sujets sous la supervision du technicien, dans les conditions suivantes :
   - Site d'application : corps.
   - Conditions d'application : application par massage digital, pour les plus de 50 ans, et par massage digital profond, en insistant au niveau des articulations, pour les sportifs.
□ Application à domicile, par les sujets, dans les conditions normales d'emploi, pendant 28 jours consécutifs.
   - Site d'application : corps, 3 fois par jour de J1 à J28.
   - Conditions d'utilisation du produit d'investigation : application par massage digital au niveau du corps pour les plus de 50 ans, et par massage digital profond, au niveau du corps en insistant au niveau des articulations, pour les sportifs, après le sport)

Vérification de l'acceptabilité cutanée (tolérance locale) basée sur :
□ un examen cutané de la zone expérimentale par le même investigateur / technicien sous la supervision de l'investigateur, au centre d'investigation, à :
   J1/T0, avant la 1ère application, J fin, après 28 jours d'utilisation (+/- 2 jours),
□ l'analyse des sensations d'inconfort rapportées directement par les participants à l'investigateur / au technicien en cours d'étude ou dans la fiche de suivi journalier.

Appréciation des qualités cosmétiques et de l'efficacité après analyse du questionnaire adapté au produit d'investigation, établi par le moniteur d'étude et rempli par chaque participant, après 28 jours consécutifs d'utilisation du produit.

Aucun signe clinique imputable au produit d'investigation n'a été observé par l'investigateur pendant l'étude. Aucune sensation d'inconfort n'a été rapportée par les sujets pendant l'étude.

### Résultats :

### Caractéristiques sensorielles : Tableau 2

**Tableau 2**

| Caractéristiques sensorielles | % de volontaires |
|---|---|
| La couleur est agréable | 90 |
| La texture est agréable | 90 |
| La texture n'est pas grasse au toucher | 80 |
| La texture n'est pas collante au toucher | 65 |
| La texture est fraîche | 95 |
| La texture est légère | 90 |
| L'aspect est agréable | 85 |
| L'odeur est agréable | 65 |

Il est relevé de plus que l'application est facile (95% des volontaires, la pénétration du produit est rapide (75% des volontaires), le produit ne tache pas les vêtements (100% des volontaires).

### Efficacité cosmétique : Tableaux 3 et 4

**Tableau 3**

| Appréciation | % de volontaires satisfaits |
|---|---|
| PEAU PLUS DOUCE | 85 |
| PEAU PLUS TONIQUE | 65 |
| LE PRODUIT PROCURE UNE SENSATION DE BIEN ETRE DURABLE | 65 |
| LE PRODUIT OFFRE UNE SENSATION IMMEDIATE DE FRAICHEUR | 75 |
| LE PRODUIT OFFRE UNE SENSATION IMMEDIATE DE LEGERETE | 80 |
| LES JAMBES SONT PLUS LEGERES | 60 |
| LES ARTICULATIONS SONT « DEVERROUILLEES « | 55 |
| SENSATIONSDE LOURDEUR SONT ATTENUEES | 55 |

**Tableau 4**

| Appréciation | % de volontaires satisfaits |
|---|---|
| Les sensations de fatigue sont diminuées | 60 |
| Les sensations d'inconfort sont atténuées | 65 |
| Les effets visibles de la rétention d'eau sont atténués | 55 |
| Les jambes et les chevilles sont moins gonflées | 40 |
| Le drainage est stimulé | 45 |
| Le produit procure une sensation de bien être, le corps est détendu | 75 |
| Le produit favorise une bonne circulation sanguine | 50 |
| Le produit est efficace sur les sensations d'échauffement | 70 |

Le produit présente une très bonne acceptabilité cutanée.

### 2) Application buccodentaire :

Nombre de sujets inclus : 20 de 25 à 67 ans (Age moyen = 47 ans) dont 19 femmes et 1 homme ; Phototype : de II à IV ; 100% ayant les gencives sensibles
- Application au centre d'investigation par les sujets sous la supervision du technicien, dans les conditions suivantes :
- Sites d'application : gencives.
- Conditions d'application : mettre une noisette de produit sur une brosse à dents souple, brosser doucement pendant 5 minutes.
- Jour d'application au centre d'investigation : J1.
- Application à domicile, par les sujets, dans les conditions normales d'emploi, pendant 28 jours consécutifs.
- Sites d'application : gencives, 2 fois par jour (matin et soir).
- Conditions d'utilisation du produit d'investigation : mettre une noisette de produit sur une brosse à dents souple, brosser doucement pendant 5 minutes.

Vérification de l'acceptabilité buccodentaire (tolérance locale) basée sur :
- un examen buccodentaire par le dentiste, au centre d'investigation, à : J1/T0 avant la 1ère application, J fin après 28 jours d'utilisation (+/- 2 jours).
- l'analyse des sensations d'inconfort rapportées directement par les participants à l'investigateur / au technicien en cours d'étude ou dans la fiche de suivi journalier.

Analyse descriptive par pourcentage de participants réactifs.

Appréciation des qualités cosmétiques et de l'efficacité après analyse du questionnaire adapté au produit d'investigation, établi par le moniteur d'étude et rempli par chaque participant, après 28 jours consécutifs d'utilisation du produit.

Paramètre d'évaluation : score d'appréciation - Analyse descriptive : pourcentage de sujets satisfaits.

### RESULTATS

### Caractéristiques sensorielles : Tableau 5

**Tableau 5**

| Appréciation | % de volontaires satisfaits |
|---|---|
| La texture est agréable | 80 |
| La consistance est satisfaisante | 80 |
| Le goût est agréable | 30 |

### Acceptabilité : Tableau 6

**Tableau 6**

| Appréciation | % de volontaires satisfaits |
|---|---|
| Le produit me convient | 50 |
| Le produit est adapté à mon type de gencives | 85 |
| Le produit est adapté à un usage fréquent | 75 |
| Le produit ne donne pas de sensations d'inconfort | 75 |
| Le produit respecte l'équilibre de la bouche | 85 |
| Le produit est doux pour l'émail de mes dents | 100 |

### Efficacité : Tableau 7

**Tableau 7**

| Appréciation | % de volontaires satisfaits |
|---|---|
| Le produit a rendu mes gencives plus fortes | 75 |
| Le produit a aidé au soulagement de mes gencives sensibles | 80 |
| L'efficacité du produit sur mes gencives est immédiate | 80 |
| L'efficacité du produit sur mes gencives est durable | 75 |
| Le produit a diminué mes saignements de gencives | 75 |
| Le produit a diminué la sensibilité des dents au contact thermique (froid ou chaud) | 85 |

### 3) Application dermatologique: sécheresse et rougeur cutanée

Nombre de sujets inclus : 21 âgés de 24 à 68 ans (Age moyen = 57 ans) de sexe féminin
- Phototype : de II à IV.
- Ayant la peau sèche au niveau du corps.
- Ayant tout type de peau au niveau du visage : peau sèche (43%, n = 9), peau normale à tendance sèche (29%, n = 6), peau mixte à tendance sèche (19%, n = 4), peau mixte à tendance grasse (10%, n = 2).
- Ayant des rougeurs cutanées au niveau du visage.
- Utilisateurs réguliers/occasionnels de produits similaires.

### Application du produit d'investigation :

□ Application au centre d'investigation par les sujets sous la supervision du technicien, dans les conditions suivantes :
   □ Sites d'application : visage et corps.
   □ Conditions d'application : application par massage digital.
   □ Jour d'application au centre d'investigation : J1.
   □ Application à domicile, par les sujets, dans les conditions normales d'emploi, 3 fois par jour, pendant 28 jours consécutifs ± 2 jours.
   □ Sites d'application : visage et corps.
   □ Conditions d'utilisation du produit d'investigation : application par massage digital, 3 fois par jour, pendant 28 jours consécutifs.

Vérification de l'acceptabilité cutanée (tolérance locale) basée sur :
□ un examen cutané des zones expérimentales par le même investigateur / technicien sous la supervision de l'investigateur, au centre d'investigation, à :
   J1/T0, avant la 1ère application,
   J fin, après 28 ± 2 jours d'utilisation,
□ l'analyse des sensations d'inconfort rapportées directement par les participants à l'investigateur / technicien en cours d'étude ou dans la fiche de suivi journalier
   Analyse descriptive par pourcentage de participants réactifs

### Evaluation de l'effet sur la sécheresse cutanée au niveau du corps (1 mollet choisi):

□ par le biais de scorage clinique à l'aide d'une échelle ordinale en 5 points de 0 à 4, par l'investigateur :
- J1/T0 avant la 1ère application.
- Jfin/T1 après 28 +/- 2 jours consécutifs d'utilisation (1 heure après la dernière application à domicile).

Paramètre d'évaluation : score clinique - Analyse descriptive et analyse statistique

### Evaluation de l'effet sur l'aspect des rougeurs cutanées au niveau du visage :

□ par le biais de scorage clinique à l'aide d'une échelle ordinale en 4 points de 0 à 3, par l'investigateur:
- J1T0 avant la 1ère application.
- JfinT1h après 28 +/- 2 jours consécutifs d'utilisation (1 heure après la dernière application à domicile).

Paramètre d'évaluation : score clinique - Analyse descriptive et analyse statistique.

Photographies numériques de chaque zone expérimentale : visage (1 face + 1 profil choisi) et corps (1 mollet choisi), au centre d'investigation, avant la première application de produit, et après 28 +/- 2 jours consécutifs d'utilisation (J1T0 et JfinT1h).

Appréciation des qualités cosmétiques et de l'efficacité après analyse du questionnaire adapté au produit d'investigation, établi par le moniteur d'étude et rempli par chaque participant, après 28+/-2 jours consécutifs d'utilisation du produit.

Paramètre d'évaluation : score d'appréciation - Analyse descriptive : pourcentage de sujets satisfaits.

### RESULTATS:

### Evaluation de l'effet sur la sécheresse cutanée au niveau du corps (mollet) (Tableau 8) :

Echelle ordinale utilisée pour le scorage clinique :
- Score 0 : absence de sécheresse (pas de squame, pas de rougeur, peau parfaitement douce et souple au toucher).
- Score 1 : sécheresse très légère (très légère desquamation à peine perceptible, pas de rougeur, peau encore souple).
- Score 2 : sécheresse légère (légère desquamation, petites squames seulement, surface légèrement terne, peau légèrement irrégulière et rugueuse lors du toucher tangentiel).
- Score 3 : sécheresse modérée (petites squames + quelques squames plus grandes, surface opaque ou blanchâtre, peau irrégulière et rugueuse lors du toucher tangentiel, légèrement ferme lors du toucher vertical, éventuellement petites zones de très légère rougeur ou faible rougeur diffuse.
- Score 4 : sécheresse sévère (petites et grandes squames uniformément distribuées, surface blanchâtre, peau très irrégulière et rugueuse, zones dures lors du toucher vertical, éventuellement zones de rougeur franches ou rougeur diffuse nette voire prononcée et une ou quelques fissures superficielles.

**Tableau 8**

| | J1T0 | JfinT1h | Variation JfinTlh- J1T0 | % Variation (JfinT1h-J1T0) /J1T0 |
|---|---|---|---|---|
| Moyenne | 2,48 | 1,52 | -0,95 | -40,5 |
| Ecart-type | 0,68 | 0,68 | 0,38 | |

### Analyse statistique : Tableau 9

Les scores de JfmT1h ont été comparés à ceux de J1T0, en utilisant le test de Wilcoxon (séries appariées).

**Tableau 9**

| | Sécheresse cutanée JfinT1h *versus* J1T0 |
|---|---|
| N21 | 21 |
| Test Wilcoxon (séries appariées) | Wilcoxon (séries appariées) |
| p | < 0,0005 |
| Interprétation statistique significatif si p<0,050 | Diminution significative |

La comparaison des scores de JfinT1h à ceux de J1T0 montre une diminution statistiquement significative de la sécheresse cutanée au niveau des mollets (de - 0.95 unités sur l'échelle de scores en 5 points (- 40,5 %)), après 29 jours consécutifs d'utilisation du produit testé au niveau du corps.

### Evaluation de l'effet sur les rougeurs cutanées au niveau du visage : Tableau 10

Echelle ordinale utilisée pour le scorage clinique :
- Score 0 : absence de rougeur visible à l'oeil nu.
- Score 1 : au moins une zone (voire plus) de rougeur très légère, rose pale, discrète mais cependant visible à l'oeil nu.
- Score 2 : au moins une zone (voire plus) de rougeur légère, rose, bien visible à l'oeil nu) et éventuellement d'une ou plusieurs zones de rougeurs très légères
- Score 3 : au moins une zone (voire plus) de rougeur modérée, rouge franc, tranchant nettement sur le tégument sain autour) (au moins une zone, voire plus) et éventuellement d'une ou plusieurs zones de rougeurs très légères et / ou légères.

**Tableau 10**

| | J1T0 | JfinT1h | Variation JfinTlh- J1T0 | % Variation (JfinT1h-J1T0) /J1T0 |
|---|---|---|---|---|
| Moyenne | 2,05 | 1,33 | -0,71 | -41,3% |
| Ecart-type | 0,59 | 0,86 | 0,46 | |

### Analyse statistique : Tableau 11

Les scores de JfinT1h ont été comparés à ceux de J1T0, en utilisant le test de Wilcoxon (séries appariées).

**Tableau 11**

| | Rougeur cutanée JfinT1h *versus* J1T0 |
|---|---|
| N21 | 21 |
| Test Wilcoxon (séries appariées) | Wilcoxon (séries appariées) |
| p | < 0,0005 |
| Interprétation statistique significatif si p<0,050 | Diminution significative |

La comparaison des scores de JfinT1h à ceux de J1T0 montre une diminution statistiquement significative de la rougeur cutanée au niveau du visage (de - 0.71 unités sur l'échelle de scores en 4 points (41,3 %)), après 29 jours consécutifs d'utilisation du produit testé au niveau du visage.

### Caractéristiques sensorielles du produit : Tableau 12

**Tableau 12**

| Caractéristiques sensorielles | La texture est agréable | La texture n'est pas grasse au toucher | La texture n'est pas collante au toucher | La texture est légère | La consistance est satisfaisante | L'aspect est agréable | L'odeur est agréable |
|---|---|---|---|---|---|---|---|
| % de volontaires | 85,7% | 95,2% | 66,7% | 90,5% | 85,7% | 76,2% | 52,4% |

### Acceptabilité cutanée : Tableau 13

**Tableau 13**

| Appréciation | % de volontaires satisfaits |
|---|---|
| Le produit me convient | 47,6 |
| Le produit ne laisse pas la peau collante | 71,4 |
| Le produit est adapté à mon type de peau | 57,1 |
| Le produit ne donne pas de sensations d'inconfort | 71,4 |
| Le produit ne dessèche pas la peau | 71,4 |

### Efficacité cosmétique : Tableau 14

**Tableau 14**

| Appréciation | % de volontaires satisfaits |
|---|---|
| La peau est plus douce | 85,7 |
| La peau est plus souple | 76,2 |
| L'intensité des rougeurs est diminuée | 61,9 |
| La peau est plus hydratée, moins desséchée | 66,7 |
| La peau est apaisée | 76,2 |
| La peau est tonifiée, raffermie | 66,7 |
| La peau est protégée des agressions extérieures (vent, froid, climatisation...) et est plus résistante | 61,9 |
| Le produit procure une sensation de bien être | 71,4 |
| Rides et ridules sont lissées | 57,1 |
| La fréquence d'apparition des sensations d'inconfort dues à l'environnement est diminuée | 71,4 |
| Le produit apaise durablement les sensations d'inconfort dues à l'environnement | 66,7 |
| Le produit apaise immédiatement les sensations d'inconfort dues à l'environnement | 71,4 |

Dans les conditions expérimentales adoptées (application répétée dans les conditions normales d'emploi, pendant 28 jours consécutifs, par un panel de 21 personnes, de sexe féminin), la composition selon l'exemple 1 présente :
- une bonne acceptabilité cutanée.
- un effet sur la sécheresse cutanée au niveau du corps statistiquement significatif (scorage clinique)
- un effet sur les rougeurs cutanées au niveau du visage statistiquement significatif (scorage clinique).

## Revendications

1. Composition sous forme de gel, **caractérisée en ce qu'**elle comprend-au moins 90 % d'eau de mer, en poids par rapport au poids total de la composition, et de l'hydroxyéthyl méthyl cellulose à titre d'agent gélifiant.

2. Composition selon la revendication 1, **caractérisée en ce qu'**elle comprend moins 95% d'eau de mer, plus préférentiellement encore au moins 98%, en poids par rapport au poids total de la composition.

3. Composition selon l'une des revendications précédentes, **caractérisée en ce que** l'eau de mer est une eau de mer filtrée et stérilisée à froid et sans radio stérilisation de façon à en conserver tous ses caractères physicochimiques et notamment les oligoéléments.

4. Composition selon l'une des revendications précédentes, **caractérisée en ce qu'**elle comprend à titre de solvant de l'eau distillée.

5. Composition selon l'une des revendications précédentes, **caractérisée en ce qu'**elle comprend au moins un agent de conditionnement de la peau tel qu'un agent hydratant, un agent bactériostatique ou fongistatique, préférentiellement choisi parmi le pentylène glycol, le caprylyl glycol, décylène glycol , ou un mélange des trois ou encore des huiles essentielles.

6. Composition selon l'une des revendications précédentes, **caractérisée en ce qu'**elle comprend entre 1 et 2%, plus préférentiellement 1,5 %, de l'agent gélifiant, en poids par rapport au poids total de la composition.

7. Composition selon l'une des revendications précédentes, **caractérisée en ce qu'**elle présente un pH compris entre 5 et 6, préférentiellement 5,5 plus ou moins 0,2 unité.

8. Composition sous forme de gel telle que définie dans l'une des revendications 1 à 7, pour son utilisation en tant que composition pharmaceutique, cosmétique ou médicament, en particulier pour son utilisation dans le traitement des traumatismes et pathologies musculo-tendineuses et en particulier, des entorses, des courbatures, des contractures musculaires, chez l'homme ou l'animal.

9. Composition sous forme de gel telle que définie dans l'une des revendications 1 à 7, pour son utilisation dans le traitement ou la prévention de la sensibilité des gencives et/ou du parodonte, en particulier dans le traitement ou la prévention des gingivites et des parodontites.

10. Composition sous forme de gel telle que définie dans l'une des revendications 1 à 7, pour son utilisation dans le traitement ou la prévention des sécheresses et/ou rougeurs cutanées du corps et/ou du visage.

## Patentansprüche

1. Gelförmige Zusammensetzung, die **dadurch gekennzeichnet ist, dass** sie mindestens 90 % Meerwasser, nach Gewicht unter Bezugnahme auf das Gesamtgewicht der Zusammensetzung, sowie Hydroxylethylmethylcellulose als Gelbildner umfasst.

2. Zusammensetzung nach Anspruch 1, **dadurch gekennzeichnet, dass** sie weniger 95 % Meerwasser, mit noch größerem Vorzug mindestens 98 % umfasst, nach Gewicht unter Bezugnahme auf das Gesamtgewicht der Zusammensetzung.

3. Zusammensetzung nach einem der vorhergehenden Ansprüche, **dadurch gekennzeichnet, dass** es sich bei dem Meerwasser um ein Meerwasser handelt, welches derart filtriert sowie kalt und ohne sterilisierte Bestrahlung sterilisiert wurde, dass sämtliche seiner physikalischchemischen Eigenschaften und insbesondere die Spurenelemente davon erhalten bleiben.

4. Zusammensetzung nach einem der vorhergehenden Ansprüche, **dadurch gekennzeichnet, dass** sie als Lösungsmittel destilliertes Wasser umfasst.

5. Zusammensetzung nach einem der vorhergehenden Ansprüche, **dadurch gekennzeichnet, dass** sie mindestens ein Mittel zum Konditionieren der Haut, wie etwa ein feuchtigkeitsspendendes Mittel, ein bakteriostatisches oder fungistatisches Mittel, welches vorzugsweise aus Pentylenglykol, Caprylylglykol, Decylenglykol oder einer Mischung der drei ausgewählt ist, oder aber ätherische Öle umfasst.

6. Zusammensetzung nach einem der vorhergehenden Ansprüche, **dadurch gekennzeichnet, dass** sie 1 bis 2 %, mit größerem Vorzug 1,5 %, des Gelbildners umfasst, nach Gewicht unter Bezugnahme auf das Gesamtgewicht der Zusammensetzung.

7. Zusammensetzung nach einem der vorhergehenden Ansprüche, **dadurch gekennzeichnet, dass** sie einen pH-Wert von 5 bis 6 aufweist, wobei dieser vorzugsweise 5,5 plus/minus 0,2 Einheiten beträgt.

8. Gelförmige Zusammensetzung nach der Begriffsbestimmung in einem der Ansprüche 1 bis 7, zur Verwendung als pharmazeutische, Kosmetikum oder Arzneimittelzusammensetzung, insbesondere zur Verwendung in der Behandlung von Verletzungen und Erkrankungen von Muskeln und Sehnen sowie insbesondere von Verstauchungen, belastungsbedingten Muskelschmerzen, Muskelkontrakturen beim Menschen und beim Tier.

9. Gelförmige Zusammensetzung nach der Begriffsbestimmung in einem der Ansprüche 1 bis 7, zur Verwendung in der Behandlung oder Verhütung von empfindlichem Zahnfleisch und/oder empfindlichem Zahnhalteapparat, insbesondere in der Behandlung oder Verhütung von Zahnfleischentzündungen und Parodontitis-Erkrankungen.

10. Gelförmige Zusammensetzung nach der Begriffsbestimmung in einem der Ansprüche 1 bis 7, zur Verwendung in der Behandlung oder Verhütung von Trockenheit und/oder Hautrötungen des Körpers und/oder des Gesichts.

## Claims

1. Composition in the gel form, **characterized in that** it comprises at least 90% of seawater, by weight with respect to the total weight of the composition, and hydroxyethyl methyl cellulose as gelling agent.

2. Composition according to Claim 1, **characterized in that** it comprises less 95% of seawater, more preferably still at least 98%, by weight with respect to the total weight of the composition.

3. Composition according to either of the preceding claims, **characterized in that** the seawater is a seawater which is filtered and sterilized under cold conditions and without radiosterilization, so as to retain all its physicochemical characteristics and in particular the trace elements thereof.

4. Composition according to one of the preceding claims, **characterized in that** it comprises, as solvent, distilled water.

5. Composition according to one of the preceding claims, **characterized in that** it comprises at least one conditioning agent for the skin, such as a moisturizing agent, a bacteriostatic or fungistatic agent, preferably chosen from pentylene glycol, caprylyl glycol or decylene glycol, or a mixture of the three or also essential oils.

6. Composition according to one of the preceding claims, **characterized in that** it comprises between 1% and 2%, more preferably 1.5%, of the gelling agent, by weight with respect to the total weight of the composition.

7. Composition according to one of the preceding claims, **characterized in that** it exhibits a pH of between 5 and 6, preferably 5.5, plus or minus 0.2 unit.

8. Composition in the gel form as defined in one of Claims 1 to 7, for its use as pharmaceutical, cosmetic or medicine composition, in particular for its use in the treatment of traumas and musculotendinous pathologies and in particular sprains, aches or cramps, in man or animals.

9. Composition in the gel form as defined in one of Claims 1 to 7, for its use in the treatment or the prevention of sensitivity of the gums and/or periodontium, in particular in the treatment or the prevention of gingivitis and periodontitis.

10. Composition in the gel form as defined in one of Claims 1 to 7, for its use in the treatment or the prevention of dryness and/or redness of the skin of the body and/or of the face.
